# EUROPEAN PATENT APPLICATION

(11) **EP 4 583 050 A1**
(43) Date of publication of application: **09.07.2025**
(21) Application number: 23870272.4
(22) Date of filing: 08.09.2023
(51) Int. Cl.: G06T 7/246, G06T 7/00, G06T 19/00

(54) **MARK POINT RECOGNITION METHOD AND APPARATUS, AND DEVICE AND STORAGE MEDIUM**

(30) Priority: 30.09.2022 CN 202211209537
(71) Applicant: Shining 3D Tech Co., Ltd., Hangzhou, Zhejiang 311258 (CN)
(72) Inventor: CHEN, Xiaojun, Hangzhou, Zhejiang 311258 (CN); ZHANG, Huiquan, Hangzhou, Zhejiang 311258 (CN); KANG, Shuaibing, Hangzhou, Zhejiang 311258 (CN); XIANG, Xiaoping, Hangzhou, Zhejiang 311258 (CN)
(74) Representative: Grünecker Patent- und Rechtsanwälte PartG mbB
(86) International application number: PCT/CN2023/117858
(87) International publication number: WO 2024/067033

(57) **Abstract**

Embodiments of the present disclosure relate to a method and apparatus for recognizing mark points, a device, and a storage medium. The method for recognizing mark points includes: acquiring a plurality of first three-dimensional coordinates corresponding to a plurality of mark points when a maxilla and a mandible are in a first pose; acquiring a plurality of second three-dimensional coordinates corresponding to the plurality of mark points when the maxilla and the mandible are in a second pose; and recognizing, according to the plurality of first three-dimensional coordinates and the plurality of second three-dimensional coordinates, first mark points located on the maxilla and second mark points located on the mandible from the plurality of mark points. According to the embodiments of the present disclosure, applicability of the mark points in mandibular movement tracking can be improved.

## Description

The present disclosure claims priority to Chinese Patent Application No. 202211209537.X, entitled "METHOD AND APPARATUS FOR RECOGNIZING MARK POINTS, DEVICE, AND STORAGE MEDIUM", filed with the China National Intellectual Property Administration on September 30, 2022, which is incorporated by reference in its entirety.

### Technical Field

Embodiments of the present disclosure relate to the field of computer technology, and in particular, to a method and apparatus for recognizing mark points, a device, and a storage medium.

### Background

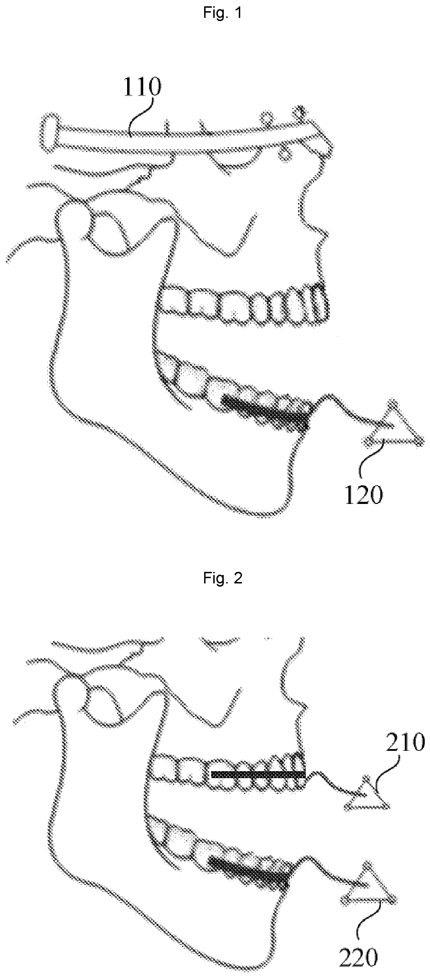

In existing technology, dynamic analysis of the mandibular movement is a crucial means for treating temporomandibular joint disorders, occlusion diseases, dentition defects, and other symptoms, and therefore research methods for mandibular movement tracking have been continuously improving with technological development. In existing mandibular movement tracking solutions, a user is often required to wear a target to track the mandibular movement by tracking the movement of mark points on the target. In some solutions, as shown in Fig. 1, the user needs to attach a maxillary target 110 to the frontal bone and a mandibular target 120 to the lower dentition. In other solutions, as shown in Fig. 2, the user needs to attach a maxillary target 210 to the upper dentition and a mandibular target 220 to the lower dentition.

However, since the shape and the structure of the target are fixed, the shape and structure may not fit well with the dental condition of the user. For example, a target cannot be fixed in the mouth of an edentulous user with severe overbite, leading to poor adaptability in clinical use.

### Summary

### (1) Technical problem to be addressed

According to the technical problem to be solved in the present disclosure, the problem that since a shape and a structure of an existing target are fixed, the shape and the structure may not fit well with a dental condition of a user, for example, a target cannot be fixed in the mouth of an edentulous user with severe overbite, leading to poor adaptability in clinical use is addressed.

### (2) Technical solutions

In order to solve the above technical problems, embodiments of the present disclosure provide a method and apparatus for recognizing mark points, a device, and a storage medium, including:
in a first aspect, embodiments of the present disclosure provide a method for recognizing mark points, including:
acquiring a plurality of first three-dimensional coordinates corresponding to a plurality of mark points when a maxilla and a mandible are in a first pose;
acquiring a plurality of second three-dimensional coordinates corresponding to the plurality of mark points when the maxilla and the mandible are in a second pose; and
recognizing, according to the plurality of first three-dimensional coordinates and the plurality of second three-dimensional coordinates, first mark points located on the maxilla and second mark points located on the mandible from the plurality of mark points.

In a second aspect, embodiments of the present disclosure provide an apparatus for recognizing mark points, including:
a first acquiring component, configured to acquire a plurality of first three-dimensional coordinates corresponding to a plurality of mark points when a maxilla and a mandible are in a first pose;
a second acquiring component, configured to acquire a plurality of second three-dimensional coordinates corresponding to the plurality of mark points when the maxilla and the mandible are in a second pose; and
a recognition component, configured to recognize, according to the plurality of first three-dimensional coordinates and the plurality of second three-dimensional coordinates, first mark points located on the maxilla and second mark points located on the mandible from the plurality of mark points.

In a third aspect, embodiments of the present disclosure provide an electronic device. The server includes a processor and a memory, where the memory has a computer program stored therein, and the computer program, when executed by the processor, causes the processor to perform the method in the first aspect.

In a fourth aspect, embodiments of the present disclosure provide a computer-readable storage medium. The storage medium has a computer program stored therein. The computer program, when executed by a processor, may implement the method for recognizing mark points in the first aspect.

### (3) Beneficial effects

Compared with the related art, the above technical solutions provided by the embodiments of the present disclosure have the following advantages:
according to the method for recognizing mark points provided in the embodiment of the present disclosure, the plurality of first three-dimensional coordinates corresponding to the plurality of mark points may be acquired when the maxilla and the mandible are in the first pose, and the plurality of second three-dimensional coordinates corresponding to the plurality of mark points are acquired when the maxilla and the mandible are in the second pose, thereby recognizing, according to the plurality of first three-dimensional coordinates and the plurality of second three-dimensional coordinates, the first mark points located on the maxilla and the second mark points located on the mandible from the plurality of mark points. By adopting the above technical solutions, since the plurality of mark points for tracking the mandibular movement may be directly fixed to the teeth or gingival mucosa of the jaw (the maxilla and the mandible), the specific fixed positions of the plurality of mark points may be flexibly selected according to the dental condition of the user in clinical use. Meanwhile, by acquiring the plurality of first three-dimensional coordinates and the plurality of second three-dimensional coordinates corresponding to the plurality of mark points, the first mark points located on the maxilla and the second mark points located on the mandible may be distinguished using a scanner, thereby facilitating subsequent application in mandibular movement tracking, and then improving the applicability of the mark points in mandibular movement tracking.

It should be understood that the above general description and the following detailed description are only exemplary and explanatory, and cannot limit the present disclosure.

### Brief Description of the Drawings

The accompanying drawings herein are incorporated into the specification to form a part of the specification, illustrate embodiments conforming to the present disclosure, and are used to explain the principle of the present disclosure together with the specification.

In order to describe the technical solutions in the embodiments of the present disclosure or in the related art more clearly, the accompanying drawings required to be used in descriptions of the embodiments or the prior art will be briefly introduced below, and it is apparent that those of ordinary skill in the art may also obtain other accompanying drawings according to these accompanying drawings without creative work.
Fig. 1 is a schematic diagram of a structure of a target according to the related art;
Fig. 2 is a schematic diagram of a structure of another target according to the related art;
Fig. 3 is a flowchart of a method for recognizing mark points according to embodiments of the present disclosure;
Fig. 4 is a schematic diagram of a first pose according to embodiments of the present disclosure;
Fig. 5 is a schematic diagram of a second pose according to embodiments of the present disclosure;
Fig. 6 is a schematic diagram of a vertical axis according to embodiments of the present disclosure;
Fig. 7 is a schematic diagram of a structure of an apparatus for recognizing mark points according to embodiments of the present disclosure; and
Fig. 8 is a schematic diagram of a structure of an electronic device according to embodiments of the present disclosure.

### Detailed Description of the Embodiments

To make objectives, technical solutions, and advantages of embodiments of the present disclosure more clear, the technical solutions in the embodiments of the present disclosure are clearly and completely described as below, and it is apparent that the described embodiments are a part rather all of embodiments of the present disclosure. Based on the embodiments of the present disclosure, all other embodiments obtained by those of ordinary skill in the art without creative work shall fall within the scope of protection of the present disclosure.

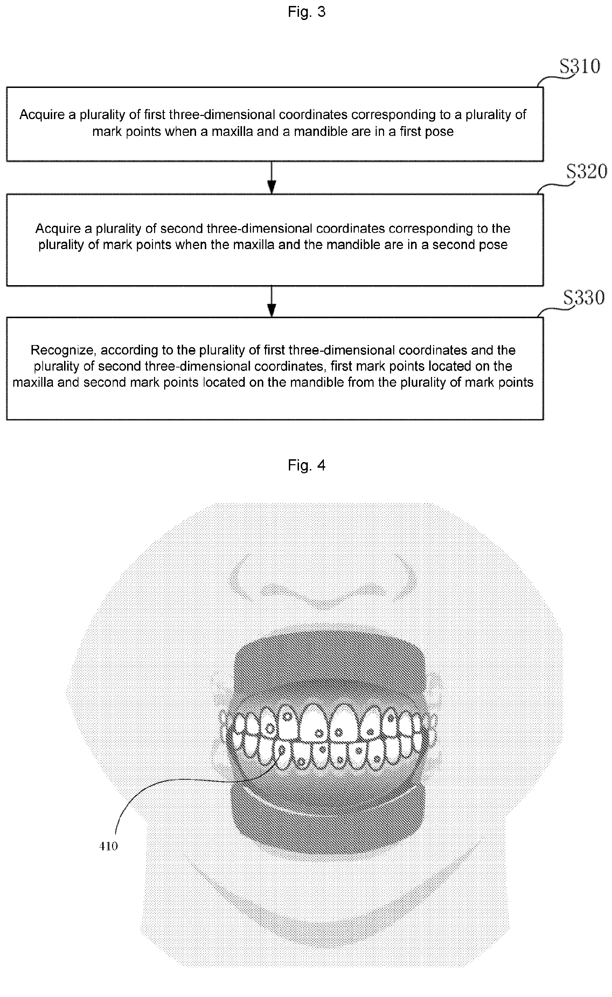

Fig. 3 is a flowchart of a method for recognizing mark points according to embodiments of the present disclosure. The method may be performed by an electronic device. The electronic device may for example, be understood as a device with a page display function, such as a smartphone, a tablet, a notebook computer, a desktop computer, and a smart television. As shown in Fig. 3, the method according to the embodiment includes the following steps:
S310: Acquire a plurality of first three-dimensional coordinates corresponding to a plurality of mark points when a maxilla and a mandible are in a first pose.

In some embodiments of the present disclosure, the plurality of mark points may be pre-fixed (e.g., fixing through methods such as adhesion) to the jaw of the user according to a dental condition of the user. Specifically, one or more first mark points are fixed to the maxilla, and one or more second mark points are fixed to the mandible. Accordingly, when the maxilla and the mandible of the user are in the first pose, the plurality of first three-dimensional coordinates corresponding to the plurality of mark points may be acquired.

In some embodiments of the present disclosure, a specific form of the mark points may be set by those skilled in the art according to actual situations, and is not limited herein.

In some embodiments of the present disclosure, from a spatial dimension, the mark points may include two-dimensional mark points or three-dimensional mark points; and from a shape, the mark points may include mark points with circular features, rectangular features, and triangular features. For example, the mark points may be two-dimensional circular mark points, three-dimensional spherical mark points, two-dimensional rectangular mark points, two-dimensional triangular mark points, etc., but are not limited thereto.

It should be noted that the plurality of mark points may be the same in size and shape. Certainly, the plurality of mark points may also include at least two forms (sizes and shapes) of mark points. For example, all the first mark points fixed to the maxilla are the same in size and shape, all the second mark points fixed to the mandible are the same in size and shape, but the sizes and/or the shapes of the first mark points may be different from those of the second mark points, but are not limited thereto.

In some embodiments of the present disclosure, the number of the mark points and specific positions on the jaw may be customized according to the dental condition of the user, which are not limited herein.

In some embodiments of the present disclosure, in the case of an edentulous jaw and severe overbite, the mark points may be fixed to the gum; and in the case that teeth exist without overbite, the mark points may be fixed to the teeth and/or gum, but are not limited thereto.

In some embodiments of the present disclosure, the first pose may be any pose that can expose the plurality of mark points.

In some embodiments of the present disclosure, Fig. 4 is a schematic diagram of a first pose according to embodiments of the present disclosure. Referring to Fig. 4, a plurality of two-dimensional circular mark points 410 adhere to the jaw of the user.

In some embodiments of the present disclosure, first three-dimensional coordinates are three-dimensional coordinates used to represent positions of the mark points in the first pose. The first three-dimensional coordinates may be coordinates in a world coordinate system or a camera coordinate system, which are not limited herein.

In some embodiments of the present disclosure, a plurality of first three-dimensional coordinates corresponding to a plurality of mark points may be acquired using a three-dimensional scanning device such as an intraoral scanner and a facial scanner. In this case, the first three-dimensional coordinates are coordinates in the camera coordinate system (i.e., a three-dimensional scanning device coordinate system), but are not limited thereto.

S320: Acquire a plurality of second three-dimensional coordinates corresponding to the plurality of mark points when the maxilla and the mandible are in a second pose.

In some embodiments of the present disclosure, when the user is guided to move the maxilla and the mandible to make the maxilla and the mandible in the second pose, the plurality of second three-dimensional coordinates corresponding to the plurality of mark points may be acquired.

In some embodiments of the present disclosure, the second pose may be any pose that can expose the plurality of mark points and is different from the first pose.

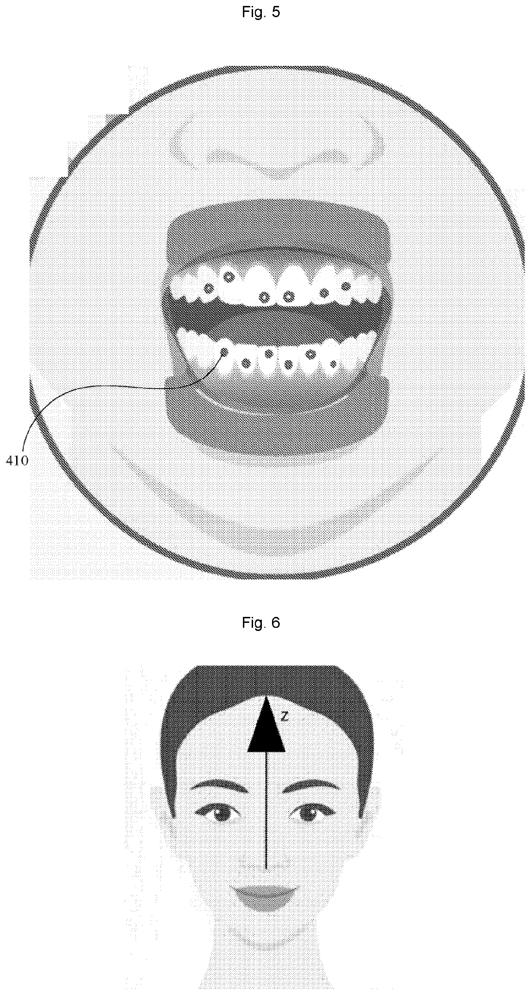

In some embodiments of the present disclosure, Fig. 5 is a schematic diagram of a second pose according to embodiments of the present disclosure. Referring to Fig. 4 and Fig. 5, since the first pose is different from the second pose, the positions of the plurality of two-dimensional circular mark points 410 on the jaw of the user change relative to the first pose.

In some embodiments of the present disclosure, the second three-dimensional coordinates are three-dimensional coordinates used to represent positions of the mark points in the second pose. The first three-dimensional coordinates may be coordinates in a world coordinate system or a camera coordinate system, which are not limited herein.

In some embodiments of the present disclosure, a plurality of second three-dimensional coordinates corresponding to a plurality of mark points may be acquired using a three-dimensional scanning device such as an intraoral scanner and a facial scanner. In this case, the second three-dimensional coordinates are coordinates in the camera coordinate system (i.e., a three-dimensional scanning device coordinate system), but are not limited thereto.

S330: Recognize, according to the plurality of first three-dimensional coordinates and the plurality of second three-dimensional coordinates, first mark points located on the maxilla and second mark points located on the mandible from the plurality of mark points.

In some embodiments of the present disclosure, after acquiring the plurality of first three-dimensional coordinates corresponding to the plurality of mark points in the first pose and the plurality of second three-dimensional coordinates corresponding to the plurality of mark points in the second pose, the plurality of mark points may be distinguished according to the plurality of first three-dimensional coordinates and the plurality of second three-dimensional coordinates, so that the first mark points located on the maxilla and the second mark points located on the mandible are distinguished, thereby facilitating subsequent tracking of the jaw movement.

It should be understood that through researches on targets in the related art, the applicant has found that targets shown in Fig. 1 have the following problems: first, in practice, during the mandibular movement, an upper target 110 fixed to the frontal bone is affected by moving skin and muscles and inevitably moves, leading to a significant error between a tracked mandibular movement relative to the maxilla and an actual mandibular movement relative to the maxilla; second, there is a relatively long distance between a lower target 120 and posterior teeth, and a transformation relationship for movement tracking is proportional to the distance, leading to an error between the tracked mandibular movement relative to the maxilla and the actual mandibular movement relative to the maxilla being further amplified by the long distance; and third, since the shape and the structure of the upper target 110 and the lower target 120 are fixed, the shape and the structure of the upper target 110 and the lower target 120 may not be fitted with the dental condition of the user, resulting in poor adaptability in clinical use. The targets shown in Fig. 2 have the same second and third problems as the targets shown in Fig. 1. In view of this, the applicant has considered directly fixing the plurality of mark points to the jaw. On one hand, since one or more first mark points may be directly fixed to the maxilla, there is no need to fix the upper target to the frontal bone, thereby avoiding errors caused by the movement of the upper target. On the other hand, since the mark points are directly fixed to the jaw and may be fixed to the posterior teeth, the distance between the mark points and the posterior teeth may be shortened, thereby reducing tracking errors. Further, since fixed positions of the mark points may be customized according to the dental condition of the user, the applicability of the mark points in mandibular movement tracking can be improved.

In some embodiments of the present disclosure, since the plurality of mark points for tracking the mandibular movement may be directly fixed to the teeth or gingival mucosa of the jaw (the maxilla and the mandible), the specific fixed positions of the plurality of mark points may be flexibly selected according to the dental condition of the user in clinical use. Meanwhile, by acquiring the plurality of first three-dimensional coordinates and the plurality of second three-dimensional coordinates corresponding to the plurality of mark points, the first mark points located on the maxilla and the second mark points located on the mandible may be distinguished, thereby facilitating subsequent application in mandibular movement tracking, and then improving the applicability of the mark points in mandibular movement tracking.

In some embodiments of the present disclosure, the step of recognizing, according to the plurality of first three-dimensional coordinates and the plurality of second three-dimensional coordinates, first mark points located on the maxilla and second mark points located on the mandible from the plurality of mark points includes:
S131: Perform concatenation processing on the plurality of mark points according to the plurality of first three-dimensional coordinates and the plurality of second three-dimensional coordinates to obtain two three-dimensional coordinate sets.

In some embodiments of the present disclosure, the concatenation processing involves determining an appropriate coordinate transformation to merge data obtained from various perspectives under a unified coordinate system. In some embodiments of the present disclosure, the concatenation processing on the plurality of mark points involves merging the plurality of first three-dimensional coordinates and the plurality of second three-dimensional coordinates under the unified coordinate system, thereby comparing the mark points in the first pose with the mark points in the second pose.

Those skilled in the art may use any possible concatenation algorithm to perform concatenation processing on the plurality of mark points, such as an iterative closest point (ICP) algorithm and a non-rigid iterative closest point (NICP) algorithm, but are not limited thereto.

In some embodiments of the present disclosure, the two three-dimensional coordinate sets are respectively a three-dimensional coordinate set corresponding to the first mark points, and a three-dimensional coordinate set corresponding to the second mark points, and three-dimensional coordinates in the two three-dimensional coordinate sets are under the same coordinate system.

The three-dimensional coordinate set corresponding to the first mark points includes: three-dimensional coordinates of each first mark point when the plurality of first three-dimensional coordinates and the plurality of second three-dimensional coordinates are merged into the unified coordinate system so that the first mark points in the first pose coincide with the first mark points in the second pose. Similarly, the three-dimensional coordinate set corresponding to the second mark points includes three-dimensional coordinates of each second mark point when the plurality of first three-dimensional coordinates and the plurality of second three-dimensional coordinates are merged into the unified coordinate system so that the second mark points in the first pose coincide with the second mark points in the second pose.

It should be understood that in the process of concatenating the plurality of mark points, when the plurality of first three-dimensional coordinates and the plurality of second three-dimensional coordinates are merged into the unified coordinate system, if the first mark points on the maxilla in the first pose coincide with the first mark points on the maxilla in the second pose, or the second mark points on the mandible in the first pose coincide with the second mark points on the mandible in the second pose, a coincidence degree of the plurality of mark points in the first pose and the plurality of mark points in the second pose is the highest. Therefore, the three-dimensional coordinates corresponding to the mark points in the two cases with the highest coincidence degree are extracted to obtain the two three-dimensional coordinate sets, and subsequently, it is only necessary to distinguish which three-dimensional coordinate set corresponds to the first mark points and which three-dimensional coordinate set corresponds to the second mark points from the two three-dimensional coordinate sets.

S132: Compare vertical axis coordinate values of the two three-dimensional coordinate sets to obtain a comparison result.

In some embodiments of the present disclosure, the coordinate system where the three-dimensional coordinates in the three-dimensional set are located includes a horizontal axis, a longitudinal axis, and a vertical axis, and the vertical axis coordinate values are coordinate values in a direction of the vertical axis, where the vertical axis represents an arrangement direction of the maxilla and the mandible.

In some embodiments of the present disclosure, Fig. 6 is a schematic diagram of a vertical axis according to embodiments of the present disclosure. Referring to Fig. 6, the maxilla and the mandible are arranged up and down in a vertical direction of paper. An extension direction of the vertical axis is a vertical direction, and a positive axis direction is upward, namely, the positive axis direction is a direction from the mandible to the maxilla.

In some embodiments of the present disclosure, S132 may include: for each three-dimensional coordinate set, averaging vertical axis coordinate values of three-dimensional coordinates in the three-dimensional coordinate set to obtain two average values corresponding to the two three-dimensional coordinate sets; and comparing the two average values, using the three-dimensional coordinate set with the smaller average value as the three-dimensional coordinate set with the smaller vertical axis coordinate values, and using the three-dimensional coordinate set with the larger average value as the three-dimensional coordinate set with the larger vertical axis coordinate values.

In some embodiments of the present disclosure, if the vertical axis is as shown in Fig. 6, one three-dimensional coordinate set (referred to as a first three-dimensional coordinate set) includes three three-dimensional coordinates, namely (x1, y1, z1), (x2, y2, z2), and (x3, y3, z3), and (z1+z2+z3)/3 is an average value corresponding to the three-dimensional coordinate set. The other three-dimensional coordinate set (referred to as a second three-dimensional coordinate set) includes (x4, y4, z4), (x5, y5, z5), and (x6, y6, z6), (z4+z5+z6)/3 is an average value corresponding to the three-dimensional coordinate set. When (z1+z2+z3)/3 is greater than (z4+z5+z6)/3, the vertical axis coordinate values of the first three-dimensional coordinate set are greater than those of the second three-dimensional coordinate set. When (z1+z2+z3)/3 is less than (z4+z5+z6)/3, the vertical axis coordinate values of the first three-dimensional coordinate set are smaller than those of the second three-dimensional coordinate set.

It should be understood that since the average value of the vertical axis coordinate values in the three-dimensional coordinate set may represent an overall magnitude of the vertical axis coordinate values in the three-dimensional coordinate set, determining an overall relationship between the vertical axis coordinate values of the two three-dimensional coordinate sets according to the average values may allow for a simple, quick, and accurate method for acquiring the comparison result.

In some other embodiments of the present disclosure, S132 may include: for each three-dimensional coordinate set, determining a maximum value from the vertical axis coordinate values of three-dimensional coordinates in the three-dimensional coordinate set to obtain two maximum values corresponding to the two three-dimensional coordinate sets; and comparing the two maximum values, using the three-dimensional coordinate set with the smaller maximum value as the three-dimensional coordinate set with the smaller vertical axis coordinate values, and using the three-dimensional coordinate set with the larger maximum value as the three-dimensional coordinate set with the larger vertical axis coordinate values.

S133: According to the comparison result, use mark points corresponding to the three-dimensional coordinates in the three-dimensional coordinate set with the larger vertical axis coordinate values as the first mark points, and use mark points corresponding to the three-dimensional coordinates in the three-dimensional coordinate set with the smaller vertical axis coordinate values as the second mark points.

Certainly, those skilled in the art should understand that if a positive axis direction of the vertical axis is a direction from the maxilla to the mandible, the mark points corresponding to the plurality of three-dimensional coordinates in the three-dimensional coordinate set with the smaller vertical axis coordinate values are used as the first mark points, and the mark points corresponding to the plurality of three-dimensional coordinates in the three-dimensional coordinate set with the larger vertical axis coordinate values are used as the second mark points.

In some embodiments of the present disclosure, the three-dimensional coordinate set corresponding to the first mark points may be denoted as a P maxillary target, the three-dimensional coordinate set corresponding to the second mark points may be denoted as a P mandibular target, and the P maxillary target and the P mandibular target are saved in a file for use during subsequent mandibular movement tracking.

In some embodiments of the present disclosure, the mark points include two-dimensional circular mark points.

It should be understood that to acquire the first three-dimensional coordinates and the second three-dimensional coordinates of the mark points, the mark points need to be first recognized from a scanning result obtained after a scanner scans the jaw. For the two-dimensional circular mark points, a circular scanning result may be obtained when scanning directly facing the mark points, an oval scanning result may be obtained when scanning not directly facing the mark points, and the oval scanning result is corrected as the circular scanning result. Then, the three-dimensional coordinates (the first three-dimensional coordinates or the second three-dimensional coordinates) are determined according to the circular scanning result. Accordingly, regardless of the direction in which the mark points are scanned, the three-dimensional coordinates may be determined through the directly obtained circular scanning result or the corrected circular scanning result, thereby obtaining more accurate three-dimensional coordinates.

In some embodiments of the present disclosure, a diameter of the two-dimensional circular mark points is less than 6 mm.

It should be understood that in the subsequent process of tracking the mandibular movement, it is often necessary to acquire jaw three-dimensional models of the jaw from different frames and concatenate the jaw three-dimensional models according to dental features such as dental fossae on the teeth. By setting the diameter of the two-dimensional circular mark points to be less than 6 mm, the problem of obscuring the dental features on the teeth due to the excessively large mark points can be avoided, thereby improving the accuracy of concatenating the jaw models.

In some embodiments of the present disclosure, the plurality of mark points may be the same in shape and size.

It should be understood that as mentioned earlier, to acquire the first three-dimensional coordinates and the second three-dimensional coordinates of the mark points, the mark points need to be first recognized from the scanning result obtained after the scanner scans the jaw. Specifically, when a certain object in the scanning result meets a preset mark point constraint condition, the object may be determined as a mark point. When the plurality of mark points are the same in shape and size, the mark point constraint condition may be set more strictly, thereby reducing the risk of misidentifying other objects as mark points.

In some embodiments of the present disclosure, the plurality of mark points include at least three first mark points that are not located on the same straight line, and at least three second mark points that are not located on the same straight line.

It should be understood that in the subsequent process of tracking the mandibular movement, it is often necessary to track the mandibular movement by tracking the movement of the mark points. In the process of tracking the movement of the mark points, three-dimensional coordinates of the mark points at a current moment and three-dimensional coordinates at a previous moment need to be concatenated. The applicant has considered that when the mark points are on the same straight line, errors are likely to occur during concatenation processing. Therefore, in some embodiments of the present disclosure, by setting the at least three first mark points that are not located on the same straight line, and the at least three second mark points that are not located on the same straight line, the risk of concatenating errors is reduced, thereby reducing the risk of jitter when calculating a mandibular movement trajectory subsequently.

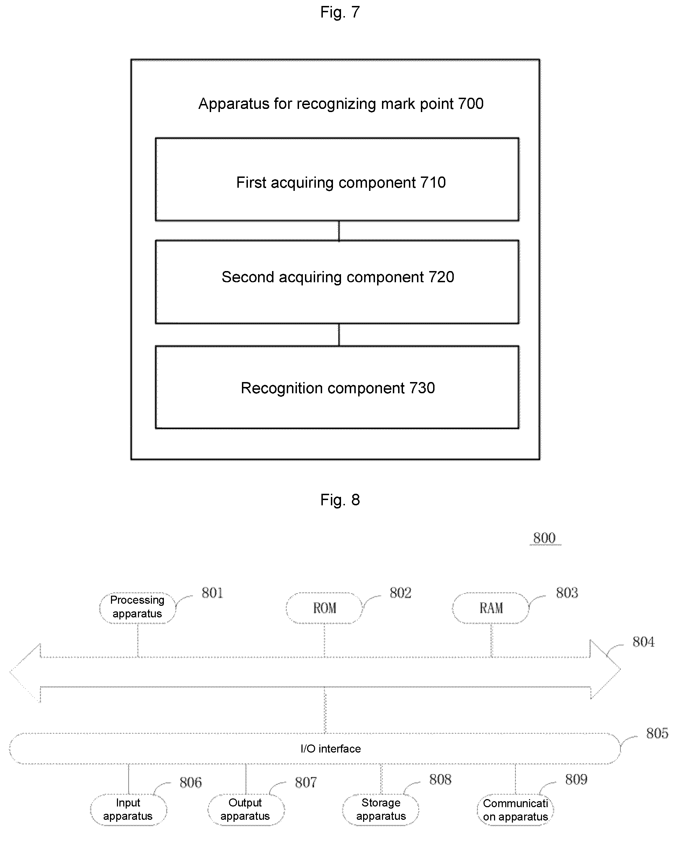

Fig. 7 is a schematic diagram of a structure of an apparatus for recognizing mark points according to embodiments of the present disclosure. The apparatus for recognizing mark points may be understood as the above electronic device or a part of functional components in the above electronic device. As shown in Fig. 7, the apparatus for recognizing mark points 700 includes:
a first acquiring component 710, configured to acquire a plurality of first three-dimensional coordinates corresponding to a plurality of mark points when a maxilla and a mandible are in a first pose;
a second acquiring component 720, configured to acquire a plurality of second three-dimensional coordinates corresponding to the plurality of mark points when the maxilla and the mandible are in a second pose; and
a recognition component 730, configured to recognize, according to the plurality of first three-dimensional coordinates and the plurality of second three-dimensional coordinates, first mark points located on the maxilla and second mark points located on the mandible from the plurality of mark points.

In some embodiments of the present disclosure, the recognition component 730 may include:
a concatenation subcomponent, configured to perform concatenation processing on the plurality of mark points according to the plurality of first three-dimensional coordinates and the plurality of second three-dimensional coordinates to obtain two three-dimensional coordinate sets;
a comparison subcomponent, configured to compare vertical axis coordinate values of the two three-dimensional coordinate sets to obtain a comparison result; and
a determination subcomponent, configured to use, according to the comparison result, mark points corresponding to three-dimensional coordinates in the three-dimensional coordinate set with the smaller vertical axis coordinate values as the first mark points, and use mark points corresponding to three-dimensional coordinates in the three-dimensional coordinate set with the larger vertical axis coordinate values as the second mark points.

In some embodiments of the present disclosure, the comparison subcomponent may include:
a first determination unit, configured to average, for each three-dimensional coordinate set, the vertical axis coordinate values of the three-dimensional coordinates in the three-dimensional coordinate set to obtain two average values corresponding to the two three-dimensional coordinate sets; and
a second determination unit, configured to compare the two average values, use the three-dimensional coordinate set with the smaller average value as the three-dimensional coordinate set with the smaller vertical axis coordinate values, and use the three-dimensional coordinate set with the larger average value as the three-dimensional coordinate set with the larger vertical axis coordinate values.

In some embodiments of the present disclosure, the mark points include two-dimensional circular mark points.

In some embodiments of the present disclosure, a diameter of the two-dimensional circular mark points is less than 6 mm.

In some embodiments of the present disclosure, the plurality of mark points are the same in shape and size.

In some embodiments of the present disclosure, the plurality of mark points include at least three first mark points that are not located on the same straight line, and at least three second mark points that are not located on the same straight line.

The apparatus according to the embodiments may perform the method according to any one of the above embodiments, with similar execution methods and beneficial effects, which will not be repeated herein.

Embodiments of the present disclosure further provide an electronic device. The electronic device includes: a memory, having a computer program stored therein; and a processor, configured to execute the computer program. The computer program, when executed by the processor, may implement the method according to any one of the above embodiments.

In some embodiments of the present disclosure, Fig. 8 is a schematic diagram of a structure of an electronic device according to embodiments of the present disclosure. Specifically, referring to Fig. 8 below, Fig. 8 illustrates a schematic diagram of a structure of an electronic device 700 suitable for implementing an embodiment of the present disclosure. The electronic device 700 in this embodiment of the present disclosure may include, but is not limited to, mobile terminals such as a mobile phone, a notebook computer, a digital radio receiver, a personal digital assistant (PDA), a portable Android device (PAD), a portable media player (PMP), and a vehicle-mounted terminal (e.g., a vehicle-mounted navigation terminal), and fixed terminals such as a digital TV and a desktop computer. The electronic device shown in Fig. 8 is merely an example, which should not impose any limitations on functions and application ranges of this embodiment of the present disclosure.

As shown in Fig. 8, the electronic device 700 may include a processing apparatus (e.g., a central processing unit and a graphics processing unit) 701 that may perform various suitable actions and processing according to a program stored in a read-only memory (ROM) 702 or a program loaded from a storage apparatus 708 into a random access memory (RAM) 703. The RAM 703 further stores various programs and data required for the operation of the electronic device 700. The processing apparatus 701, the ROM 702, and the RAM 703 are connected to one another through a bus 704. An input/output (I/O) interface 705 is also connected to the bus 704.

Typically, the following apparatuses may be connected to the I/O interface 705: an input apparatus 706 including, for example, a touch screen, a touch pad, a keyboard, a mouse, a camera, a microphone, an accelerometer, and a gyroscope; an output apparatus 707 including, for example, a liquid crystal display (LCD), a speaker, and a vibrator; the storage apparatus 708 including, for example, a magnetic tape and a hard drive; and a communication apparatus 709. The communication apparatus 709 may allow the electronic device 700 to be in wireless or wired communication with other devices for data exchange. Although Fig. 8 illustrates the electronic device 700 with various apparatuses, it should be understood that it is not necessary to implement or have all the shown apparatuses. It may be an alternative to implement or have more or fewer apparatuses.

In particular, the above process described with reference to the flowcharts according to the embodiments of the present disclosure may be implemented as a computer software program. For example, embodiments of the present disclosure include a computer program product, which includes a computer program carried on a non-transitory computer-readable medium, where the computer program includes program code for performing the method shown in the flowchart. In this embodiment, the computer program may be downloaded and installed from the network through the communication apparatus 709, installed from the storage apparatus 708, or installed from the ROM 702. The computer program, when executed by the processing apparatus 701, performs the above functions limited in the method according to the embodiments of the present disclosure.

It should be noted that the above computer-readable medium in the present disclosure may be either a computer-readable signal medium or a computer-readable storage medium, or any combination of the two. The computer-readable storage medium may be, for example, but is not limited to, electric, magnetic, optical, electromagnetic, infrared, or semiconductor systems, apparatuses, or devices, or any combination of the above. More specific examples of the computer-readable storage medium may include but not limited to: an electrical connection with one or more wires, a portable computer disk, a hard drive, a random access memory (RAM), a read-only memory (ROM), an erasable programmable read-only memory (EPROM or flash memory), an optical fiber, a portable compact disc read-only memory (CD-ROM), an optical storage device, a magnetic storage device, or any proper combination of the above. In the present disclosure, the computer-readable storage medium may be any tangible medium including or storing a program, and the program may be for use by or for use in combination with an instruction execution system, apparatus, or device. However, in the present disclosure, the computer-readable signal medium may include a data signal propagated in a baseband or as a part of a carrier, where the data signal carries computer-readable program code. The propagated data signal may take various forms, including but not limited to, an electromagnetic signal, an optical signal, or any suitable combination of the above. The computer-readable signal medium may further be any computer-readable medium other than the computer-readable storage medium. The computer-readable signal medium may send, propagate, or transmit a program for use by or for use in combination with the instruction execution system, apparatus, or device. The program code included in the computer-readable medium may be transmitted by any suitable medium, including but not limited to a wire, an optical cable, radio frequency (RF), etc., or any suitable combination of the above.

In some embodiments of the present disclosure, a client and a server may communicate using any currently known or future-developed network protocols such as a hypertext transfer protocol (HTTP), and may be interconnected with digital data communication in any form or medium (e.g., a communication network). Examples of the communication network include a local area network ("LAN"), a wide area network ("WAN"), an internetwork (e.g., the Internet), a peer-to-peer network (e.g., an ad hoc peer-to-peer network), and any currently known or future-developed network.

The above computer-readable medium may be included in the above electronic device; or may also separately exist without being assembled in the electronic device.

The above computer-readable medium carries one or more programs. The above one or more programs, when executed by the electronic device, cause the electronic device to:
acquire a plurality of first three-dimensional coordinates corresponding to a plurality of mark points when a maxilla and a mandible are in a first pose;
acquire a plurality of second three-dimensional coordinates corresponding to the plurality of mark points when the maxilla and the mandible are in a second pose; and
recognize, according to the plurality of first three-dimensional coordinates and the plurality of second three-dimensional coordinates, first mark points located on the maxilla and second mark points located on the mandible from the plurality of mark points.

Computer program code for performing operations of the present disclosure may be written in one or more programming languages or a combination thereof, where the above programming languages include, but are not limited to, object-oriented programming languages, such as Java, Smalltalk, and C++, and further include conventional procedural programming languages, such as "C" language or similar programming languages. The program code may be executed entirely on a user computer, partly on the user computer, as a stand-alone software package, partly on the user computer and partly on a remote computer, or entirely on the remote computer or the server. In the case of the remote computer, the remote computer may be connected to the user computer through any kind of network, including a local area network (LAN) or a wide area network (WAN), or may be connected to an external computer (e.g., connected through the Internet with the aid of an Internet service provider).

The flowchart and the block diagram in the accompanying drawings illustrate the possibly implemented system architecture, functions, and operations of the system, the method, and the computer program product according to various embodiments of the present disclosure. In this regard, each block in the flowchart or the block diagram may represent a component, a program segment, or a part of code, and the component, the program segment, or the part of code contains one or more executable instructions for implementing specified logical functions. It should also be noted that in some alternative implementations, the functions marked in the blocks may also occur in an order different from that marked in the accompanying drawings. For example, two blocks shown in succession may actually be performed substantially in parallel, or may sometimes be performed in a reverse order, depending on the functions involved. It should also be noted that each block in the block diagrams and/or the flowcharts, and a combination of the blocks in the block diagrams and/or the flowcharts may be implemented using a dedicated hardware-based system that performs specified functions or operations, or may be implemented using a combination of dedicated hardware and computer instructions.

The involved units described in the embodiments of the present disclosure may be implemented through software or hardware. The name of the unit does not limit the unit itself in certain cases.

Herein, the functions described above may be at least partially executed by one or more hardware logic components. For example, without limitation, exemplary hardware logic components that can be used include: a field-programmable gate array (FPGA), an application specific integrated circuit (ASIC), an application specific standard product (ASSP), a system on chip (SOC), a complex programmable logic device (CPLD), etc.

In the context of the present disclosure, a machine-readable medium may be a tangible medium that may include or store a program for use by or for use in combination with the instruction execution system, apparatus, or device. The machine-readable medium may be a machine-readable signal medium or a machine-readable storage medium. The machine-readable medium may include, but is not limited to, an electronic, magnetic, optical, electromagnetic, infrared, or semiconductor system, apparatus, or device, or any suitable combination of the above content. More specific examples of the machine-readable storage medium may include an electrical connection based on one or more wires, a portable computer disk, a hard drive, a random access memory (RAM), a read-only memory (ROM), an erasable programmable read-only memory (EPROM or a flash memory), an optical fiber, a portable compact disc read-only memory (CD-ROM), an optical storage device, a magnetic storage device, or any suitable combination of the above content.

Embodiments of the present disclosure further provide a computer-readable storage medium. The storage medium has a computer program stored therein. The computer program, when executed by a processor, may implement the method according to any one of the above embodiments, with similar execution methods and beneficial effects, which will not be repeated herein.

It should be noted that herein, relational terms such as "first" and "second" are used only to distinguish one entity or operation from another and do not necessarily require or imply any actual relationship or order between these entities or operations. In addition, the terms "comprise", "include", or any other variations thereof are intended to cover non-exclusive inclusions, and therefore a process, a method, an article, or a device including a series of elements not only includes those elements but also includes other elements not clearly listed, or further includes elements inherent to the process, the method, the article, or the device. In the absence of more restrictions, an element defined by the phrase "including a ..." does not exclude another identical element in the process, the method, the article, or the device that includes the element.

The above contents are merely specific implementations of the present disclosure, such that those skilled in the art can understand or implement the present disclosure. More modifications for these embodiments are apparent to those skilled in the art, and general principles defined herein may be implemented in other embodiments without departing from the spirit or scope of the present disclosure. Therefore, the present disclosure will not be limited by these embodiments described herein but is required to conform to a widest scope consistent with the principles and novel characteristics disclosed herein.

### Industrial applicability

According to the method for recognzing mark point provided in the present disclosure, since the plurality of mark points for tracking the mandibular movement may be directly fixed to the teeth or gingival mucosa of the jaw (the maxilla and the mandible), the specific fixed positions of the plurality of mark points may be flexibly selected according to the dental condition of the user in clinical use. Meanwhile, by acquiring the plurality of first three-dimensional coordinates and the plurality of second three-dimensional coordinates corresponding to the plurality of mark points, the first mark points located on the maxilla and the second mark points located on the mandible may be distinguished, thereby facilitating subsequent application in mandibular movement tracking, and then improving the applicability of the mark points in mandibular movement tracking.

## Claims

1. A method for recognizing mark points, comprising:
acquiring a plurality of first three-dimensional coordinates corresponding to a plurality of mark points when a maxilla and a mandible are in a first pose;
acquiring a plurality of second three-dimensional coordinates corresponding to the plurality of mark points when the maxilla and the mandible are in a second pose; and
recognizing, according to the plurality of first three-dimensional coordinates and the plurality of second three-dimensional coordinates, first mark points located on the maxilla and second mark points located on the mandible from the plurality of mark points.

2. The method as claimed in claim 1, wherein the recognizing, according to the plurality of first three-dimensional coordinates and the plurality of second three-dimensional coordinates, first mark points located on the maxilla and second mark points located on the mandible from the plurality of mark points comprises:
performing concatenation processing on the plurality of mark points according to the plurality of first three-dimensional coordinates and the plurality of second three-dimensional coordinates to obtain two three-dimensional coordinate sets;
comparing vertical axis coordinate values of the two three-dimensional coordinate sets to obtain a comparison result; and
using, according to the comparison result, mark points corresponding to three-dimensional coordinates in the three-dimensional coordinate set with larger vertical axis coordinate values as the first mark points, and using mark points corresponding to three-dimensional coordinates in the three-dimensional coordinate set with smaller vertical axis coordinate values as the second mark points.

3. The method as claimed in claim 2, wherein the comparing vertical axis coordinate values of the two three-dimensional coordinate sets to obtain the comparison result comprises:
averaging, for each three-dimensional coordinate set, the vertical axis coordinate values of the three-dimensional coordinates in the three-dimensional coordinate set to obtain two average values corresponding to the two three-dimensional coordinate sets; and
comparing the two average values, using the three-dimensional coordinate set with a smaller average value as the three-dimensional coordinate set with the smaller vertical axis coordinate values, and using the three-dimensional coordinate set with a larger average value as the three-dimensional coordinate set with the larger vertical axis coordinate values.

4. The method as claimed in any one of claims 1 to 3, wherein the mark points comprise two-dimensional circular mark points.

5. The method as claimed in claim 4, wherein a diameter of the two-dimensional circular mark points is less than 6 mm.

6. The method as claimed in any one of claims 1 to 3, wherein the plurality of mark points are the same in shape and size.

7. The method as claimed in claim 1, wherein the plurality of mark points comprise at least three first mark points that are not located on a same straight line, and at least three second mark points that are not located on the same straight line.

8. An apparatus for recognizing mark points, comprising:
a first acquiring component, configured to acquire a plurality of first three-dimensional coordinates corresponding to a plurality of mark points when a maxilla and a mandible are in a first pose;
a second acquiring component, configured to acquire a plurality of second three-dimensional coordinates corresponding to the plurality of mark points when the maxilla and the mandible are in a second pose; and
a recognition component, configured to recognize, according to the plurality of first three-dimensional coordinates and the plurality of second three-dimensional coordinates, first mark points located on the maxilla and second mark points located on the mandible from the plurality of mark points.

9. An electronic device, comprising:
a processor and a memory, wherein the memory stores a computer program, and the computer program, when executed by the processor, causes the processor to perform the method as claimed in any one of claims 1 to 7.

10. A computer-readable storage medium, wherein the storage medium stores a computer program, and the computer program, when executed by a processor, implements the method as claimed in any one of claims 1 to 7.
